**Europäisches Patentamt**

**European Patent Office**

**Office européen des brevets**

(11) Veröffentlichungsnummer: **0 446 804 A2**

(12) # EUROPÄISCHE PATENTANMELDUNG

(21) Anmeldenummer: 91103575.6

(51) Int. Cl.⁵: **A61M 25/06, A61B 17/34**

(22) Anmeldetag: 08.03.91

(30) Priorität: 16.03.90 DE 4008391

(43) Veröffentlichungstag der Anmeldung:
18.09.91 Patentblatt 91/38

(84) Benannte Vertragsstaaten:
AT BE CH DE DK ES FR GB GR IT LI NL SE

(71) Anmelder: **VYGON GMBH & CO KG**
**Goebbelgasse 100**
**W-5100 Aachen(DE)**

(72) Erfinder: **Heiliger, Raymund, Dr.**
**Schweyerstrasse 16**
**W-5120 Herzogenrath(DE)**

(74) Vertreter: **Bauer, Hubert, Dipl.-Ing.**
**Am Keilbusch 4**
**W-5100 Aachen(DE)**

(54) **Arterielles Punktionsbesteck.**

(57) Um bei einem arteriellen Punktionsbesteck einen flexiblen Draht (7) ohne aufwendige Betätigungsmittel in ein Behältnis ein- und ausbringen zu können und die Sterilität des flexiblen Drahtes (7) gewährleisten zu können, ist das Behältnis aus einem Folienschlauch (8) gebildet, der an seinem offenen distalen Ende mit einem im wesentlichen zylindrischen Kupplungsstück (9) versehen ist. An einem zylindrischen Ansatzstück (2) einer Einführungsnadel (1) ist der Folienschlauch (8) mit dem Kupplungsstück (9) anschließbar.

Fig. 1

EP 0 446 804 A2

Die Erfindung betrifft ein arterielles Punktionsbesteck aus einem flexiblen Draht, einer hohlen Einführungsnadel mit einem im wesentlichen zylindrischen Ansatzstück am proximalen Ende, einer elastischen, die Nadel umschließenden Kanüle und einem mit dem Ansatzstück der Einführungsnadel kuppelbaren an seinem proximalen Ende geschlossenen Behältnis zur Aufnahme des flexiblen Drahtes.

Ein derartiges Punktionsbesteck ist aus der EP O 093 164 B1 bekannt. Bei dem bekannten Punktionsbesteck besteht das Behältnis zur Aufnahme des flexiblen Drahtes aus einem rohrförmigen Element. Um den darin befindlichen flexiblen Draht durch die Einführungsnadel hindurch in ein Blutgefäß vorschieben und auch wieder zurückziehen zu können, ist das rohrförmige Behältnis mit einem über seine Länge reichenden Schlitz versehen, durch den am proximalen Ende des flexiblen Drahtes angreifende Betätigungseinrichtungen hindurch nach außen führen. Diese Einrichtungen bestehen aus einer auf das Drahtendstück kraftschlüssig aufgebrachten Hülse, von der eine Speiche durch den Schlitz des rohrförmigen Behältnisses nach außen führt. Das radiale Ende der Speiche geht in einen Ring über, dessen Innendurchmesser dem Außendurchmesser des rohrförmigen Behältnisses entspricht. Der Ring wird somit durch den Mantel des Behältnisses geführt und ist über dessen Länge verschiebbar. Zur Erleichterung des Bewegungsvorganges ist am Ring zusätzlich noch eine radial abstehende Fahne angeformt, die sich bequem mit zwei Fingerkuppen erfassen läßt.

Das bekannte Punktionsbesteck erfordert für die Verschiebebewegung des flexiblen Drahtes die vorbeschriebenen verhältnismäßig aufwendigen Betätigungseinrichtungen. Ein noch wesentlicherer Nachteil dieses Punktionsbesteckes ergibt sich durch den Längsschlitz des rohrförmigen Behältnisses. Dadurch kann nämlich die Sterilität des aus dem Behältnis in das Blutgefäß einzuführenden Drahtes erheblich beeinträchtigt werden. Dieser ist nämlich über seine volle Länge durch den Schlitz einer permanenten Infizierungsgefahr ausgesetzt. Diese ergibt sich in erhöhtem Ausmaß, wenn der flexible Draht aus dem Blutgefäß herausgezogen ist und vorübergehend in dem Behältnis aufbewahrt wird, um später erneut in das Blutgefäß eingeführt zu werden. Der dann zwangsläufig mit Blut oder anderen Körperflüssigkeiten benetzte Draht neigt in diesem Zustand verstärkt dazu, Stäube und pathogene Bakterien an sich zu binden, die über den Längsschlitz des Behältnisses eingedrungen und sich an der Innenwandung des Behältnisses abgelagert haben können. Trotz der aufwendigen Drahtführung läßt sich nämlich nicht verhindern, daß der flexible Draht zumindest streckenweise an der Innenwandung des Behältnisses scheuert und dadurch im erhöhten Maße kontaminiert wird.

Der Erfindung liegt die Aufgabe zugrunde, ein Punktionsbesteck der eingangs beschriebenen Art so auszubilden, daß sich der flexible Draht ohne aufwendige Betätigungsmittel in ein Behältnis ein- und ausbringen läßt, dessen Sterilität zu gewährleisten ist.

Zur Lösung dieser Aufgabe wird von einem Punktionsbesteck der im Oberbegriff des Anspruchs 1 genannten gattungsgemäßen Art ausgegangen, welches erfindungsgemäß die im kennzeichnenden Teil desselben angegebenen Merkmale aufweist.

Das erfindungsgemäß aus einem Folienschlauch bestehende Behältnis ist nicht nur außerordentlich kostengünstig herzustellen, sondern erlaubt es, auf jegliche Betätigugngsmittel für die Verschiebebewegung des Drahtes zu verzichten, und kann insbesondere vollständig geschlossen ausgebildet werden. Durch die Flexibilität des Folienschlauches kann der Draht, soweit er sich im Behältnis befindet, an jeder Stelle durch zwei Fingerkuppen erfaßt und beispielsweise unter Querfaltenbildung des Folienschlauches vorgeschoben und unter Aufhebung der Falten auch wieder zurückgezogen werden. Durchbrüche im Folienschlauch für speziell auszubildende Betätigungsmittel sind also ebenso wie diese selbst entbehrlich.

Der Folienschlauch weist vorzugsweise zwei flach aufeinander legbare Seitenwände auf, die in der Nähe des proximalen Endes dicht miteinander verschweißt sind.

Ein aus einem derartigen Folienschlauch gebildetes Behältnis ist in besonders einfacher Weise aus endlosem Folienschlauchmaterial zu konfektionieren.

Um die Einführungsnadel einerseits und das den flexiblen Draht einschließende Behältnis mit diesem andererseits beispielsweise zum Zweck einer nachträglichen erneuten Einführung des Drahtes in das Blutgefäß zwischenzeitlich voneinander trennen zu können und im gekuppelten Zustand der Teile das distale Ende des flexiblen Drahtes mühelos in die Einführungsnadel einbringen zu können, sieht eine weitere Ausgestaltung der Erfindung vor, daß das zylindrische Kupplungsstück mit einem außen leicht konischen Endteil unter Klemmsitz in das innen leicht konische Ansatzstück der Einführungsnadel einpreßbar ist und dabei deren proximales Ende umschließt.

Nach einer weiteren Ausgestaltung der Erfindung ist das Kupplungsstück mit dem Folienschlauch durch eine elastische Manschette dicht verbunden. Diese Verbindung ist einfach herzustellen, wobei als Manschette beispielsweise ein kurzes Gummischlauchstück verwendbar ist, das sich unter Vorspannung auf den mit seinem offenen Ende auf das Kupplungsstück aufgeschobenen Folienschlauch aufbringen läßt.

Schließlich sieht eine Ausgestaltung der Erfindung noch vor, daß der flexible Draht an seinem dem Folienschlauch zugewandten Ende mit einer den minimalen lichten Querschnitt des Kupplungsstückes überschreitenden Verdickung versehen ist. Durch diese Verdickung läßt sich verhindern, daß der flexible Draht unbeabsichtigt vollständig außerhalb des Folienschlauches gelangt und sodann über den Folienschlauch nicht mehr erfaßbar wäre.

In der Zeichnung ist ein Ausführungsbeispiel des erfindungsgemäßen Punktionsbesteckes dargestellt. Es zeigen:

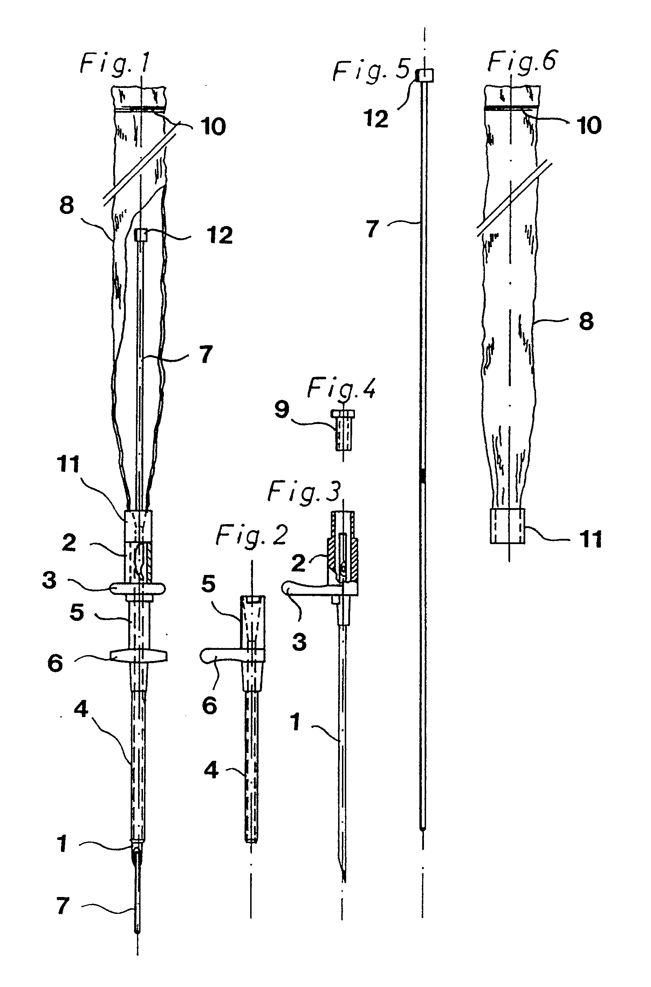

Fig. 1    das vollständige Punktionsbesteck in einer Seitenansicht, wobei das Behältnis unterbrochen und teilweise in einem Längsschnitt dargestellt ist;

Fig. 2    eine Kunststoffkanüle mit einem Ansatzstück in einer Seitenansicht;

Fig. 3    eine Einführungsnadel in einer Seitenansicht mit einem teilweise im Längsschnitt dargestellten Ansatzstück;

Fig. 4    ein Kupplungsstück für das Behältnis in einer Seitenansicht;

Fig. 5    einen flexiblen Draht in gestreckter Form;

Fig. 6    ein unterbrochen dargestelltes Behältnis in Seitenansicht.

Das Punktionsbesteck besteht aus einer konventionellen, hohl ausgebildeten Einführungsnadel 1 mit einem im wesentlichen zylindrischen Ansatzstück 2, von dem eine Griffplatte 3 radial absteht.

Eine auf die Einführungsnadel 1 aufgeschobene Kunststoffkanüle 4 ist gleichfalls mit einem im wesentlichen zylindrischen Ansatzstück 5 versehen, von dem ebenso eine Griffplatte 6 radial absteht.

Durch die Ansatzstücke 2 und 5 und durch die Einführungsnadel 1 ist ein flexibler Draht 7 hindurchgeführt, dessen distales Ende je nach seiner Position die Spitze der Einführungsnadel 1 mehr oder weniger überragen kann.

Der Draht 7 befindet sich mit seinem jeweils übrigen, das proximale Ende des Ansatzstückes 2 überragenden Teil in einem radial geschlossenen, aus einem Folienschlauch 8 gebildeten Behältnis. Dazu ist der Folienschlauch 8 über ein zylindrisches Kupplungsstück 9 mit dem Ansatzstück 2 der Einführungsnadel 1 dicht verbindbar, während sein dem Kupplungsstück gegenüberliegendes Ende durch eine Schweißnaht 10 verschlossen ist.

Die Verbindung des Folienschlauches 8 mit dem Kupplungsstück 9 erfolgt durch eine elastische Manschette 11, die unter Spannung einen Kragen des Folienschlauches 8 gegen die Außenseite des Kupplungsstückes 9 ringsum anpreßt.

Das im Folienschlauch 8 befindliche Ende des Drahtes 7 ist mit einer Verdickung 12 versehen, deren Durchmesser den minimalen Durchgangsquerschnitt des Kupplungsstückes 9 überschreitet.

**Patentansprüche**

1. Arterielles Punktionsbesteck, bestehend aus einem flexiblen Draht, einer hohlen Einführungsnadel mit einem im wesentlichen zylindrischen Ansatzstück am proximalen Ende, einer elastischen, die Nadel umschließenden Kanüle und einem mit dem Ansatzstück der Einführungsnadel verbundenen an seinem proximalen Ende geschlossenen Behältnis zur Aufnahme des flexiblen Drahtes, dadurch gekennzeichnet, daß das Behältnis aus einem Folienschlauch (8) besteht, der an seinem offenen distalen Ende mit einem im wesentlichen zylindrischen Kupplungsstück (9) versehen ist und mit diesem dicht an das zylindrische Ansatzstück (2) der Einführungsnadel (1) anschließbar ist.

2. Punktionsbesteck nach Anspruch 1, dadurch gekennzeichnet, daß der Folienschlauch (8) zwei flach aufeinander legbare Seitenwände aufweist, die in der Nähe des proximalen Endes durch eine Schweißnaht (10) dicht miteinander verbunden sind.

3. Punktionsbesteck nach Anspruch 1 oder 2, dadurch gekennzeichnet, daß das zylindrische Kupplungsstück (9) mit einem außen leicht konischen Endteil unter Klemmsitz in das innen leicht konische Ansatzstück (2) der Einführungsnadel (1) einpreßbar ist und dabei deren proximales Ende umschließt.

4. Punktionsbesteck nach mindestens einem der Ansprüche 1 bis 3, dadurch gekennzeichnet, daß das Kupplungsstück (9) mit dem Folienschlauch (8) durch eine elastische Manschette (11) dicht verbunden ist.

5. Punktionsbesteck nach mindestens einem der Ansprüche 1 bis 4, dadurch gekennzeichnet, daß der flexible Draht (7) an seinem dem Folienschlauch (8) zugewandten Ende mit einer den minimalen lichten Querschnitt des Kupplungsstückes (9) überschreitenden Verdickung (12) versehen ist.

Fig.1
Fig.2
Fig.3
Fig.4
Fig.5
Fig.6